# EUROPEAN PATENT APPLICATION

(11) **EP 2 918 229 A1**
(43) Date of publication of application: **16.09.2015**
(21) Application number: 13853026.6
(22) Date of filing: 31.10.2013
(51) Int. Cl.: A61B 6/14

(54) **IMPLANT IMAGE CREATING METHOD AND IMPLANT IMAGE CREATING SYSTEM**

(30) Priority: 08.11.2012 KR 20120126259; 04.02.2013 KR 20130012588
(71) Applicant: Megagen Implant Co., Ltd., Gyeongsangbuk-do 712-852 (KR)
(72) Inventor: PARK, Kwang Bum, Daegu 706-776 (KR)
(74) Representative: Cabinet Chaillot
(86) International application number: PCT/KR2013/009762
(87) International publication number: WO 2014/073818

(57) **Abstract**

Disclosed herein are a method and system for generating an implant image. The system includes a computed tomography (CT) data input module, a CT data correction module, and a hybrid image generation module. The CT data input module acquires CT data acquired when a bite tool provided with a reference plate is inserted into the oral cavity of a patient and stereo lithography (STL) data generated using plaster patterns of teeth. The CT data correction module makes the reference plate of the CT data identical with the reference plate of the STL data, and corrects the CT data based on the STL data. The hybrid image generation module generates a hybrid image by combining the corrected CT data with the STL structure so that the CT data represents the teeth of the patient and the STL data represents the gums of the patient.

## Description

### BACKGROUND OF THE INVENTION

### 1. Field of the Invention

The present invention relates generally to a method and system for generating an implant image and, more particularly, to a method and system for generating an implant image, which are capable of combining stereo lithography (STL) data with computed tomography (CT) data using a reference plate, thereby accurately representing the structures of the teeth and the gums.

### 2. Description of the Related Art

In general, a CT scanner is capable of acquiring a three-dimensional (3D) image of the inside of a patient using an X-ray radiation apparatus that rotates around the patient, unlike an X-ray imaging machine capable of acquiring a two-dimensional (2D) plane image. For example, a CT scanner may acquire 2D tomographic images of the body of a patient, and may generate 3D images by combining the tomographic images.

Based on blood among tissues that constitute the body, a CT scanner represents tissues having higher densities to be darker, and represents tissues having lower densities to be brighter. Accordingly, when a prosthetic appliance or prosthesis is present inside the body, X rays are scattered or diffracted, and thus an error may occur in a 2D or 3D image acquired by a CT scanner. Furthermore, since a CT scanner generates a 2D or 3D image of a region of interest among the tissues of the body, it may be somewhat inconvenient to scan all the tissues of the body.

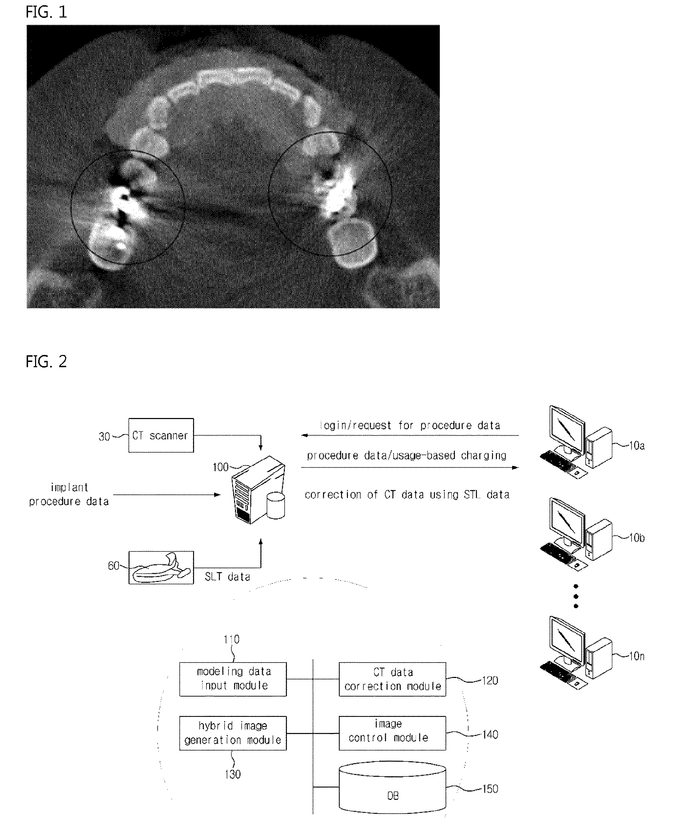

In order to overcome the above problem, Korean Patent Application No. 10-2008-0129545 discloses a 3D image acquisition apparatus that successively captures a subject (for example, a patient) using a CT scanner and generates a 2D or 3D image by combining acquired CT images with a panoramic capturing method. The 3D image acquisition apparatus disclosed in Korean Patent Application No. 10-2008-0129545 varies the distance to an X-ray light source that projects X rays onto a region of interest when X rays are projected along a trajectory to a region of interest of a subject, thereby acquiring a 3D image using the varying difference. However, there is still a problem in which an image acquired by a CT scanner is distorted by various types of prosthetic appliances and prostheses inside the body of a patient. For example, if there is a gold-capped tooth among the teeth of a patient or there are prosthetic appliances used to correct irregular teeth, X rays projected onto the teeth from a CT scanner are dispersed and diffracted by the gold-capped tooth or prosthetic appliances, thereby distorting a surrounding image. Image distortion caused by a prosthetic appliance or prosthesis is illustrated in FIG. 1. An artificial tooth illustrated in FIG. 1 reflects X rays in a diffused manner or diffracts X rays, thereby distributing an image around the artificial tooth or making the image difficult to identify. This phenomenon occurs in connection with a prosthetic appliance or prosthesis located inside the body of a patient in the same manner, which may reduce the accuracy of an image that is acquired by a CT scanner.

This may cause an error in a location where an implant will be placed in implant surgery that requires highly accurate location information in order to place the implant. Furthermore, if a prosthetic appliance or prosthesis is present near a target tooth, there is concern that a 2D or 3D image acquired by an expensive CT scanner may become useless.

### SUMMARY OF THE INVENTION

Accordingly, the present invention has been made keeping in mind the above problems occurring in the prior art, and an object of the present invention is to provide a method and system for generating an implant image, which are capable of combining the advantages of STL data with the advantages of CT data, thereby generating an accurate 3D image of the structures of the teeth and gums of the patient and also enabling the accurate and safe placement of a dental implant to be performed using the accurate 3D image.

In accordance with an aspect of the present invention, there is provided a method of generating an implant image, the method being performed by a system for generating an implant image that generates an implant image and displays the implant image on a display device, the method including acquiring CT data acquired when a bite tool provided with a reference plate is inserted into the oral cavity of a patient and STL data configured to represent a 3D object using polygons while referring to plaster patterns of the teeth; making coordinates of a reference plate of the CT data identical with coordinates of a reference plate of the STL data, and correcting the CT data based on the STL data; and generating a hybrid image so that the corrected CT data represents a first density region and the STL data represents a second density region.

In accordance with another aspect of the present invention, there is provided a system for generating an implant image, including a CT data input module configured to acquire CT data acquired when a bite tool provided with a reference plate is inserted into the oral cavity of a patient and STL data generated using plaster patterns of teeth; a CT data correction module configured to make a reference plate of the CT data identical with a reference plate of the STL data, and to correct the CT data based on the STL data; and a hybrid image generation module configured to generate a hybrid image by combining the corrected CT data with the STL structure so that the CT data represents the teeth of the patient and the STL data represents the gums of the patient, thereby allowing the CT and STL data to represent different density regions, respectively.

### ADVANTAGEOUS EFFECTS

In accordance with the present invention, accurate modeling data related to the structures of the teeth and the gums is generated by combining CT data with STL data using the reference plate, and a hybrid image in which the teeth and the gums are clearly represented is generated using the generated modeling data and then used. Furthermore, dentists who place implants can prevent surgical accidents by performing accurate placement using hybrid images according to the present invention, and contribute to the development of medicine by sharing the results of the placement of implants.

### BRIEF DESCRIPTION OF THE DRAWINGS

FIG. 1 is a photo illustrating an example in which a CT image has been distorted by a prosthetic appliance or prosthesis;
FIG. 2 is a conceptual diagram of an a system for generating an implant image according to an embodiment of the present invention;
FIG. 3 is a diagram illustrating an example of a bite tool that is inserted into the oral cavity of a patient;
FIG. 4 is a flowchart of a method of generating an implant image according to an embodiment of the present invention;
FIG. 5 is a flowchart of a method of controlling a hybrid image according to an embodiment of the present invention;
FIGS. 6 and 7 are screen shots illustrating an example of displaying a panoramic image on a screen;
FIG. 8 is a photo showing a trial product of a bite tool that was manufactured by the present applicant;
FIGS. 9 and 10 are conceptual diagrams illustrating an example in which the system for generating an implant image corrects the coordinate information of STL and CT data; and
FIG. 11 is a diagram illustrating an example of a hybrid image that is generated by combining a CT image and an STL image.

### DESCRIPTION OF THE PREFERRED EMBODIMENTS

A bite tool that is described herein is inserted into the oral cavity of a patient. The bite tool may be used to model the structures of the teeth and the gums inside the oral cavity of a patient. The bite tool may be provided with a reference plate. The reference plate may be provided to extend from one side of a bite tool to the outside of the oral cavity of a patient. The reference plate may be used to correct CT data.

Plaster patterns that are described herein may be formed by printing the shapes of the teeth and the gums on plaster. Plaster is disposed inside a bite tool, and enables the shapes of the teeth and the gums around the teeth to be printed thereon when the bite tool is inserted into the oral cavity of a patient and then the teeth of the patient press the plaster.

STL data that is described herein may be configured in ASCII or binary form. STL data may refer to data that represents the surfaces of a 3D object using polygons so that the modeling data of a 3D object can be easily recognized by different types of 3D programs.

CT data that is described herein may refer to data about tomographic images of the teeth of a patient that are captured by a CT scanner. Alternatively, although CT data may refer to a 3D image that is generated using a plurality of tomographic images, the CT data is not limited thereto.

Embodiments of the present invention will be described in detail below with reference to the accompanying drawings.

FIG. 2 is a conceptual diagram of a system 100 for generating an implant image according to an embodiment of the present invention.

Referring to FIG. 2, the system 100 for generating an implant image according to this embodiment of the present invention is connected to a plurality of user terminals 10a to 10n over a network, and is connected to a CT scanner 30 via a wired or wireless connection and can thus acquire CT data from the CT scanner 30. In this case, the CT scanner 30 may generate CT data by tomographically scanning the structures of the skull and teeth of a patient and the gums around the teeth using X rays.

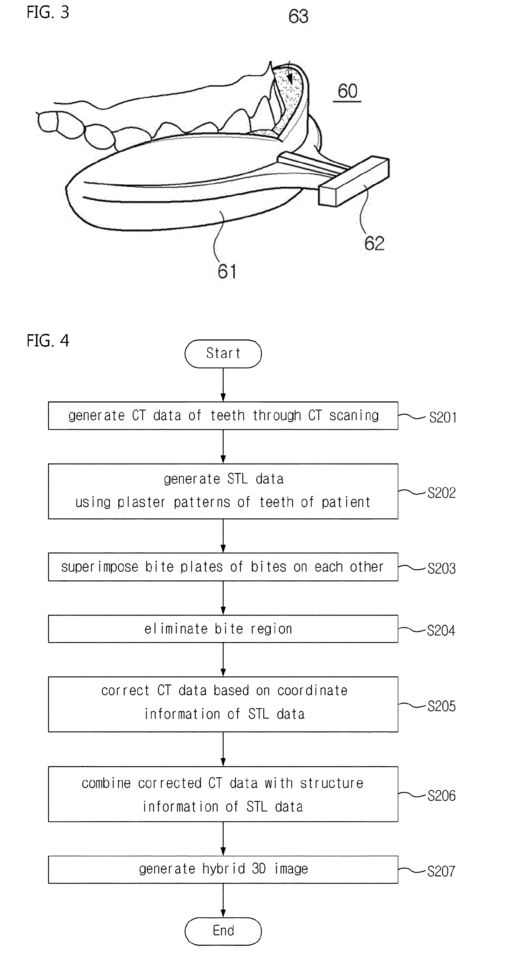

The CT data may include not only information about the structures and densities of the teeth and the gums but also data about a bite tool 60. The bite tool 60 is inserted into the oral cavity of the patient. When the bite tool 60 is engaged with the teeth, the bite tool 60 is filled with plaster used to make patterns for the structure of the teeth and the gums around the teeth, and may be formed such that a reference plate extends in a direction opposite the direction of insertion into the oral cavity. The structure of the bite tool 60 will be described with additional reference to FIG. 3.

FIG. 3 is a diagram illustrating an example of a bite tool that is inserted into the oral cavity of a patient.

The illustrated bite tool 60 may include a body 61 configured to have a curvature along the arrangement of the teeth of the patient, and a reference plate 62 configured to extend from the center of the front teeth of the patient in a direction opposite that of the oral cavity. The bite tool 63 is configured such that the space of the bite tool 60 that faces the teeth of the patient is filled with plaster 63 and the structure of the teeth of the patient and the structure of the gums around the teeth are printed on the plaster 63 when the filling plaster 63 is pressed by the teeth.

The reference plate 62 provided in the bite tool 60 has an approximately square pillar shape, and may be formed to protrude from the center of the bite tool 60 to the outside of the teeth of the patient. Information about the reference plate 62 is not only included in the CT data, but is also included in STL data that is generated with respect to plaster patterns after the plaster patterns of the teeth and gums of the patient have been made. That is, data about the reference plate 62 may be included in both the CT data and the STL data.

The CT data and STL data in which the data about the bite tool 60 is included is provided to the system 100 for generating an implant image according to an embodiment of the present invention, and the system 100 for generating an implant image may correct the CT data based on the STL data that is acquired via the plaster patterns and that has accurate dimensions. For this purpose, the system 100 for generating an implant image may make the coordinate information of the reference plate 62 in the STL data identical with the coordinate information of the reference plate 62 in the CT data. The system 100 for generating an implant image:
(1) corrects the coordinate information of the reference plate 62 included in the CT data into the coordinate information of the reference plate 62 included in the STL data. This may be referred to as "reference point correction."
(2) after the coordinate information of the reference plate 62 has been corrected in the CT data, corrects the coordinates of the teeth based on the corrected coordinates of the reference plate 62.
(3) allows the distances between the coordinates of the CT data to be resized based on the correction of the coordinates of the CT data.
(4) after the CT data has been corrected, generates a hybrid image by combining the corrected CT data with the STL data. A hybrid image is generated by combining data about exoskeleton structures, such as the structure of the teeth and the structure of the gums, in the STL data with the CT data in which the coordinates have been corrected, thereby enabling the CT data incapable of representing the gums to faithfully represent the structure of the gums.

As described in (1) to (4), the system 100 for generating an implant image may generate a hybrid image, and may display the generated hybrid image on a display device. The display device may be one of devices, such as an LCD, an LED, a PDP and a CRT, that is connected to the system 100 for generating an implant image.

Meanwhile, the system 100 for generating an implant image may provide implant procedure data to the user terminals 10a to 10n of a hospital, a public health center, a medical school and other organizations that require knowledge and experience related to an implant procedure.

The implant procedure data may be configured in the form of a moving image in which a user interface that is provided by the system 100 for generating an implant image has been applied to an actual implant procedure. Furthermore, the implant procedure data may be learning materials related to the user interface itself that is provided by the system 100 for generating an implant image. The implant procedure data may be charged for according to a usage-based charging system based on a period from the time at which one of the user terminals 10a to 10n logs in to the system 100 for generating an implant image to the time at which a connection is terminated. Alternatively, the implant procedure data may be charged for in proportion to a period in which one of the user terminals 10a to 10n has accessed the implant procedure data. However, a data price charging system related to the implant procedure data is not limited thereto.

Preferably, the system 100 for generating an implant image may include a modeling data input module 110, a CT data correction module 120, a hybrid image generation module 130, an image control module 140, and a database 150.

After the bite tool 60 has been inserted into the oral cavity of a patient, the modeling data input module 110 may input CT data, that is, data acquired by the CT scanner 30, and STL data, that is, 3D modeling data related to the bite tool 60 inserted into the oral cavity of the patient and plaster patterns acquired using the bite tool 60.

The 3D modeling data may be generated by scanning the bite tool 60, the reference plate 62 and the plaster patterns using a 3D scanner. In this case, the generated 3D modeling data may be the STL data. Since the STL data is free from external influences imposed by a prosthetic appliance and/or a prosthesis that may exist within the oral cavity of a patient or factors that interrupt 3D scanning, it can most accurately represent the teeth and gums of the patient.

After the CT data and the STL data have been acquired, the modeling data input module 110 may provide the data to the CT data correction module 120.

The CT data correction module 120 extracts the coordinate information of the reference plate 62 included in the CT data and the coordinate information of the reference plate 62 included in the STL data, and corrects the coordinate information of the reference plate 62 included in the CT data to the coordinate information of the reference plate 62 included in the STL data. The CT data may be corrected using any of the following methods:
(5) a method of performing correction by applying corrected differences to the overall coordinate information of the CT data when the coordinate information of the reference plate 62 included in the CT data is corrected; and
(6) a method of correcting the distances between the reference plate 62, the teeth and the gums using the values of the STL data based on corrected coordinate information after the coordinate information of the reference plate 62 included in the CT data has been corrected.

For example, assuming that the distance between the coordinate information of the reference plate 62 included in the CT data and a molar tooth is L1 and the distance between the reference plate 62 and the molar tooth included in the STL data is L2, the CT data correction module 120 corrects the coordinate information of the reference plate 62 included in the CT data to coordinate information in the STL data, and then corrects the distance between the reference plate 62 and the molar tooth in the corrected CT data to L2. Through this process, the overall CT data may be corrected based on the STL data.

Thereafter, the CT data correction module 120 may finally correct the CT data by resizing the differences between individual coordinates that constitute the 3D image.

The resized CT data may be provided to the hybrid image generation module 130, and the hybrid image generation module 130 may generate a hybrid image in which the teeth and the gums are all represented by combining the corrected CT data with the exoskeleton information of the STL data.

The structures of the gums printed on the plaster patterns via a 3D scanner may be represented using STL data that are represented by means of polygons, such as triangles, rectangles, pentagons, and other polygons. Accordingly, when STL data that has the same size as corrected CT data is combined with CT data, the CT data may represent all of the teeth, the oral cavity and the gums. In this case, since the coordinates and size of the CT data have been corrected based on the STL data, the corrected CT data can be corrected with accuracy similar to the accuracy of the STL data.

FIG. 4 is a flowchart of a method of generating an implant image according to an embodiment of the present invention. FIG. 4 will be described in conjunction with FIGS. 2 and 3.

Referring to FIG. 4, first, the oral cavity of a patient into which the bite tool 60 has been inserted is captured using the CT scanner 30 and CT data is acquired via the CT scanner 30 at step S201. Thereafter, the bite tool 60 is removed from the oral cavity of the patient, plaster patterns are made for the structures of the teeth and gums of the patient via the removed bite tool 60, and STL data, that is, 3D modeling data, is generated using the plaster patterns at step S202. In this case, data about the bite tool 60 may be included in the CT data and the STL data. Furthermore, the STL data may be acquired through the 3D scanning of the plaster patterns. Data about the location and structure of the bite tool 60 is included in the STL data and the CT data.

Thereafter, the system 100 for generating an implant image superimposes the bite tool 60 included in the CT data on the bite tool 60 included in the STL data at step S203. The superimposition of the bite tools 60 on each other may be performed using any of a method of performing superimposition via the images of the CT data and the STL data and a method of superimposing the coordinate information of the bite tool 60 in the CT data on the coordinate information of the bite tool 60 in the STL data.

The superimposition of the bite tools 60 of the CT data and the STL data on each other means that the reference points of the CT data and the STL data, that is, the locations of the two bite tools 60, are set to the same point. Through this setting, the CT data and the STL data may be made to share the same reference point, and also the coordinate information of the CT data may be corrected in accordance with the STL data.

Thereafter, the system 100 for generating an implant image removes data about the bite tools 60 from the CT data and the STL data at step S204, and leaves only data about the teeth, gums and oral cavity of the patient. After the data about the bite tools 60 has been removed, the system 100 for generating an implant image may correct the coordinate information of the CT data in which the location information of the reference plate 62 has been corrected while referring to the STL data at step S205. In the correction of the coordinate information, the coordinate information of the CT data may be performed by applying the distances between the reference plate 62 used as a reference point and the teeth included in the STL data to the CT data.

For example, assuming that the distance between the reference plate 62 and the molar tooth is D1 in the STL data and the distance between the reference plate 62 and the molar tooth is D2 in the CT data, D2 may be corrected to D1. Through this correction, coordinate information included in the CT data may be corrected by referring to the distances between the reference plate 62 and the coordinate information of the STL data. After the coordinate information of the CT data has been corrected, the system 100 for generating an implant image combined the corrected CT data with the STL data and may add the exoskeleton structure of the STL data to the CT data at step S206.

The STL data includes an exoskeleton structure about the teeth and the gums around the teeth. When the STL data is combined with CT data in the state in which the CT data has been corrected such that images of the same size are represented, the structure of the gums that cannot be represented by the CT data may be represented based on the CT data.

In the case of CT data, blood or the gums having low density have lower resolution than the teeth because X-rays are radiated onto the teeth and gums of the patient and then represent density differences. In contrast, STL data can clearly represent the structures of the teeth and the gums around the teeth because it is 3D modeling data based on the plaster patterns of the structures of the teeth and gums of the patient.

When the coordinate information of CT data is corrected with reference to STL data, an image represented via the CT data and an image via the STL data can be resized to the same size. Accordingly, the CT data and the STL data, which are the same size images, may be superimposed on each other, the density of the teeth and the structure of the oral cavity may be desirably represented by the superimposed CT data and the STL data, and the gums located around the teeth in the STL data may be added to the image of the CT data. An image generated by combining the CT data with the STL data as described above may be referred to as a "hybrid image." The system 100 for generating an implant image generates a 3D hybrid image at step S207, and may display the 3D hybrid image on the display device, or may store 3D hybrid image in the form of a file and then provide it to the user terminals 10a to 10n.

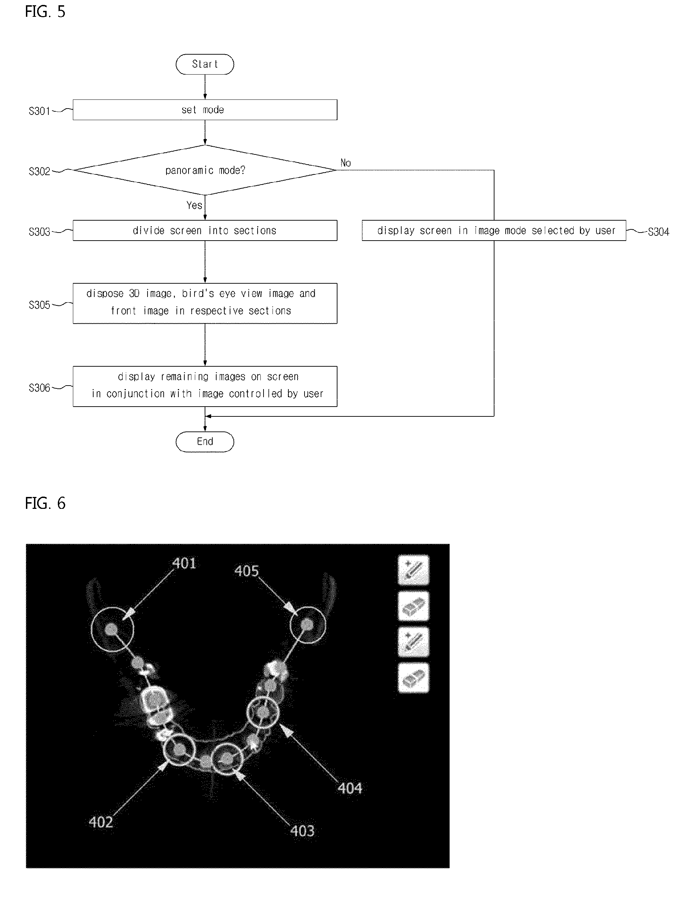

FIG. 5 is a flowchart of a method of controlling a hybrid image according to an embodiment of the present invention.

Referring to FIG. 5, first, the system 100 for generating an implant image displays a main menu on the display device, displays a user mode menu and recognizes a mode set by a user at step S301. The system 100 for generating an implant image may provide various user modes with respect to the generated hybrid image.

For example, the user modes may include an individual mode in which one of a bird's eye view image acquired by viewing the teeth from the direction of the skull, a bird's eye view image acquired by viewing the teeth from the direction of the jaws, a 3D image configured such that the skull and the teeth are represented together, and a 2D front image acquired by viewing the upper teeth and the lower teeth from the front is included, and a panoramic mode in which at least two or all of a bird's eye view image, a 3D image, and a front image are included.

Thereafter, the system 100 for generating an implant image determines whether a panorama menu option has been selected from a main menu at step S302.

If the individual mode related to one of the bird's eye view image, the 3D image and the front image, instead of the panoramic mode, has been selected by the user, the image corresponding to the individual mode may be displayed on the display device. In contrast, if the mode selected by the user is panoramic mode, the system 100 for generating an implant image may enable a bird's eye view image, a 3D image and a front image to be displayed together on a single screen, or may enable a bird's eye view image and a 3D image, or a 3D image and a front image to be displayed together on a single screen. The panoramic mode enables various images of the teeth to be displayed together on a single screen, thereby enabling a medical team to view and analyze the images of the teeth and their surrounding regions at various angles.

For this purpose, the system 100 for generating an implant image may divide a screen into sections at step S303, may dispose a 3D image, a front image and a bird's eye view image in the respective sections at step S305, and may perform control so that the 3D image, the front image and the bird's eye view image disposed in the respective sections operate in conjunction with each other, thereby displaying the images on the screen at step S306. For example, assuming that the user clicks a canine tooth in an image of the teeth represented on the front image using a mouse or a keyboard and drags the canine tooth in the direction of a molar tooth, a region where the canine tooth is represented is allowed to be displayed at the center of the screen of the 3D image when the canine tooth is selected, and a region where the molar tooth is represented is allowed to be represented on the center of the screen if the canine tooth is dragged in the direction of the molar tooth later.

Through this conjunctive operation of the images, the user may not only use the bird's eye view image, the front image and the 3D image individually, but may also enable associated regions to be represented. Meanwhile, if the mode selected by the user is not panoramic mode, the system 100 for generating an implant image enables any one of the bird's eye view image, the front image and the 3D image to be independently displayed on the screen at step S304. That is, the user enables the image corresponding to the image mode selected by the user to be displayed on the screen.

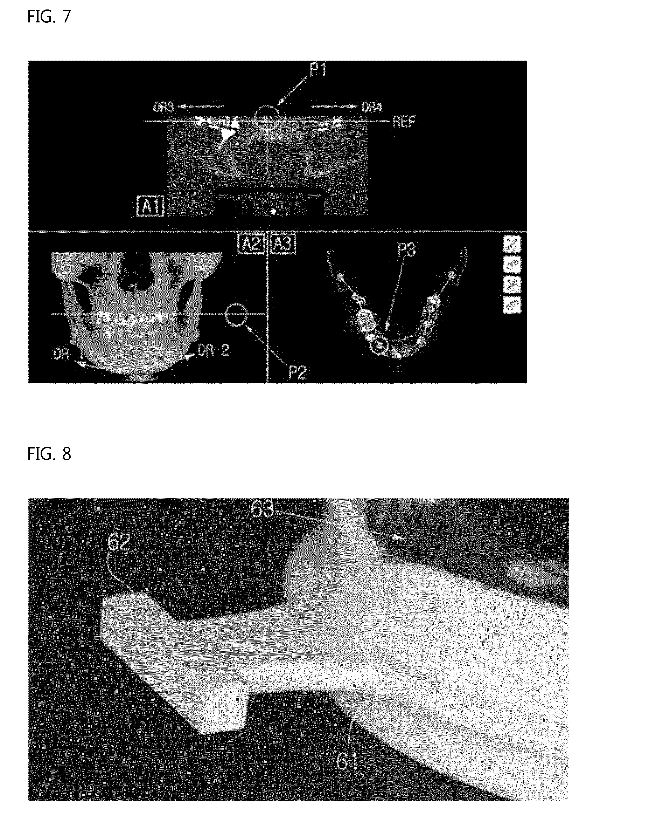

FIGS. 6 and 7 are screen shots illustrating an example of displaying a panoramic image on a screen.

Referring to FIGS. 6 and 7, FIG. 6 illustrates an example of a bird's eye view image. That is, FIG. 6 illustrates an example of a bird's eye view image that is acquired when viewed in a direction from the skull to the jaws. From the illustrated bird's eye view image, one of the teeth of the patient that a user desires to observe may be selected using an input device, such as a mouse. In FIG. 6, reference numerals 401 to 405 designate points that are selected by the user using an input device, such as a mouse. FIG. 6 illustrates an example in which the user selects a tooth that the user desires to view via 3D and front images.

Thereafter, FIG. 7 illustrates an example of images that are viewed in panoramic mode. Referring to FIG. 7, a single screen is divided into three sections A1, A2 and A3, and front, 3D and bird's eye view images are displayed on the sections A1, A2 and A3, respectively. If a user clicks point P1 on the front image displayed in section A1 and then drags a mouse in direction DR3, a 3D image displayed in section A2 may be rotated in direction DR1. If the user clicks point P1 on the front image displayed in section A1 and drags the mouse in direction DR4, a 3D image displayed in section A2 may be rotated in direction DR4.

That is, if the user selects a desired location on the front image displayed in section A1 using an input device, such as a mouse and a drag input is applied in the state, a 3D image displayed in section A2 may be also rotated in direction DR3 or DR4 in order to display an associated location. In this case, a bird's eye view image displayed in section A3 shows a circular diagram at a location selected from the front image, thus enabling the user to synthetically determine the states and structures of the teeth of the patient and the gums around the teeth while referring to the bird's eye view image, 3D image and front image together.

Meanwhile, in FIG. 7, line P2 may correspond to reference line REF of the teeth on the front image displayed in section A1.

Furthermore, on the bird's eye view of image section A3, a location that the user desires to observe corresponds to point P3, and the location of point P3 corresponds to that of point PI. That is, this means that the images of sections A1, A2 and A3 operate in conjunction with each other so that the location of the same tooth can be located at the center of a screen.

FIG. 8 is a photo showing a trial product of a bite tool that was manufactured by the present applicant.

Referring to FIG. 8, the bite tool may include a body 61 configured to be inserted into the oral cavity of a patient, a reference plate 62, and plaster 63.

The body 61 and the reference plate 62 may be made of plastic. The reference plate 62 may be integrated with the body 61, and may protrude from the body 61 so that it can be located outside the oral cavity. Through this configuration, the reference plate 62 may be prevented from being twisted or bent inside the oral cavity, in which state the CT and STL data of the reference plate 62 may be generated.

The plaster 63 may be provided at a location that faces the teeth of the patient. When the patient applies pressure to the plaster 63 using his or her teeth, the shapes of the teeth of the patient and the gums around the teeth may be printed on the plaster 63. The printed shapes of the teeth and the gums may be used for a 3D scanner to generate STL data later.

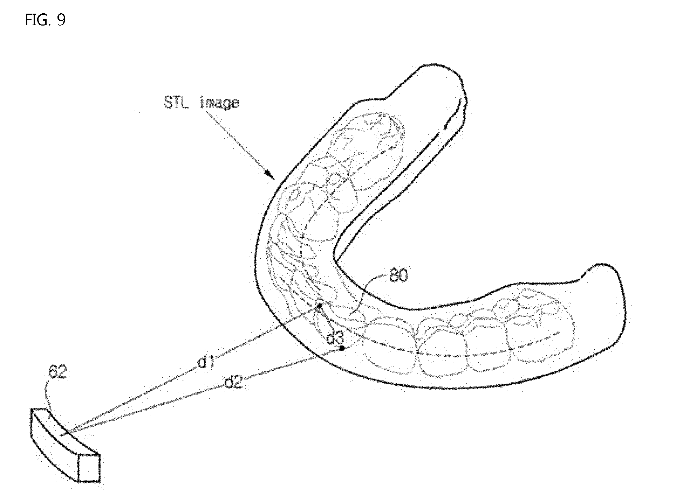

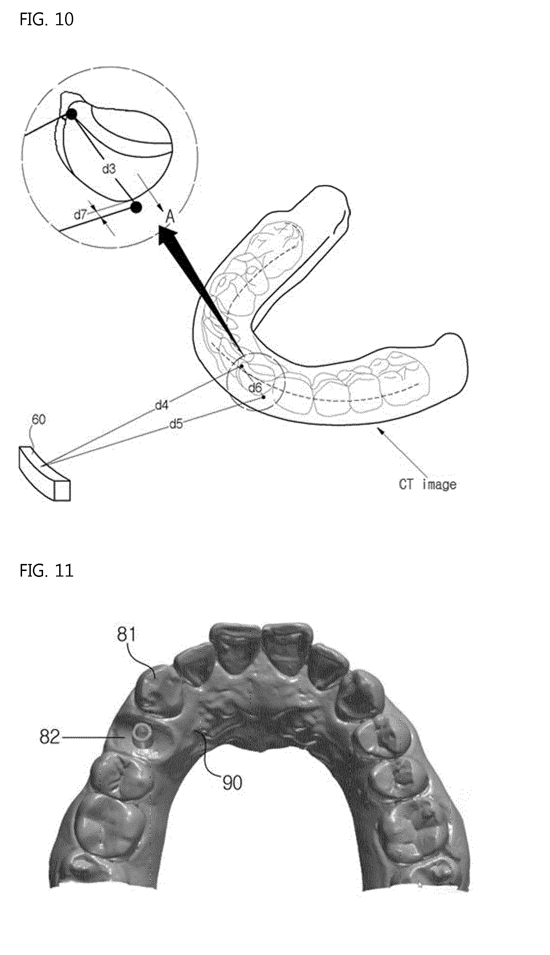

FIGS. 9 and 10 are conceptual diagrams illustrating an example in which the system for generating an implant image corrects the coordinate information of STL and CT data.

Referring to FIGS. 9 and 10 together, an STL image generated based on STL data and a CT image generated based on CT data enables distances and directions related to the teeth to be defined based on the reference plate 62. The reference plate 62 illustrated in FIGS. 9 and 10 are the same.

Before FIGS. 9 and 10 are compared with each other, the images of FIGS. 9 and 10 will be described below.

### - Descriptions -

- In FIG. 9, the distances between the reference plate 62 and both sides of a tooth 80 are d1 and d2, respectively.
- In FIG. 10, the distances between the reference plate 62 and both side ends of a tooth 81 are d4 and d5, respectively.
- A CT image illustrated in FIG. 10 corresponds to a CT image that is generated based on CT data whose coordinate information has not been corrected.
- On the CT image illustrated in FIG. 10, distance d3 between both side ends of the tooth 81 has been offset by distance d7 in direction A of one side of the tooth 81. This directional offset, that is, a type of CT image error, may result from image distortion that is generated by a prosthetic appliance or prosthesis that is located around the tooth 80.

In this case, if distances d4 and d5 are corrected using d1 and d2, that is, the distances between the reference plate 62 and the tooth 80, d4 and d5, that is, the distances between the reference plate 62 and the teeth 81 illustrated in FIG. 10, may be corrected to d1 and d2, respectively. In this manner, the distances between the reference plate 62 and each of the teeth of a patient may be corrected by correcting the values of the CT data while referring those defined in the STL data.

In the present invention, the reference plate 62 may be a reference point based on which distances and directions related to each tooth are corrected in the CT data.

Accordingly, it is necessary to make the reference plate 62 of the STL data identical with the reference plate 62 of the CT data. Before the coordinate information of the CT data is corrected, the coordinate information of the reference plate 62 should be corrected in accordance with the STL data. After the coordinate information of the reference plate 62 has been corrected in the CT data, the coordinate information of the teeth included in the CT data may be corrected based on the STL data. Accordingly, the shape and coordinate information of each of the teeth represented in the CT data may be resized based on the STL data. After the resizing has been processed, CT and STL images generated based on the CT and STL data may have the same size. The shapes and densities of the teeth and the gums may be represented by combining the CT image with the exoskeleton structures of the gums represented on the STL image. An image in which the shapes of the teeth and the gums have been represented through the combination of the CT and STL data is referred to as a hybrid image, which may generated in a form that is illustrated in FIG. 11.

## Claims

1. A method of generating an implant image, the method being performed by a system for generating an implant image that generates an implant image and displays the implant image on a display device, the method comprising:
acquiring computed tomography (CT) data acquired when a bite tool provided with a reference plate is inserted into an oral cavity of a patient and stereo lithography (STL) data configured to represent a three-dimensional (3D) object using polygons while referring to plaster patterns of teeth;
making coordinates of a reference plate of the CT data identical with coordinates of a reference plate of the STL data, and correcting the CT data based on the STL data; and
generating a hybrid image so that the corrected CT data represents a first density region and the STL data represents a second density region.

2. The method of claim 1, wherein correcting the CT data comprises correcting coordinate information of the CT data to coordinate information of the STL data.

3. The method of claim 1, wherein correcting the CT data comprises resizing the CT data so that a size of the CT data fits that of the STL data.

4. The method of claim 1, wherein correcting the CT data comprises correcting coordinate information of the reference plate provided in the CT data to coordinate information of the reference plate provided in the STL data, and correcting coordinate information of the CT data based on the corrected coordinate information of the reference plate.

5. The method of claim 1, wherein the hybrid image is an image that is generated by incorporating an exoskeleton structure of the STL data into the CT data.

6. The method of claim 1, further comprising, after acquiring the CT data and the STL data, making the reference plate of the CT data identical with the reference plate of the STL data, and eliminating image data related to the reference plate and the bite tool.

7. The method of claim 1, further comprising, after generating the hybrid image;
dividing a display area of a screen into a first section and a second section;
disposing plane and 3D images of the teeth in the first and second sections, respectively; and
displaying a conjunctive image on the screen, the conjunctive image being an image in which, when user control is applied to any one image of the plane and 3D images, a remaining image changes in conjunction with the any one image.

8. The method of claim 7, wherein the conjunctive image is a rotating image.

9. A system for generating an implant image, comprising:
a CT data input module configured to acquire CT data acquired when a bite tool provided with a reference plate is inserted into an oral cavity of a patient and STL data generated using plaster patterns of teeth;
a CT data correction module configured to make a reference plate of the CT data identical with a reference plate of the STL data, and to correct the CT data based on the STL data; and
a hybrid image generation module configured to generate a hybrid image by combining the corrected CT data with the STL structure so that the CT data represents teeth of the patient and the STL data represents gums of the patient, thereby allowing the CT and STL data to represent different density regions, respectively.

10. The system of claim 9, wherein the CT data correction module performs reference point correction in which coordinates of the reference plate provided in the CT data are corrected to coordinates of the reference plate provided in the STL data.

11. The system of claim 10, wherein the CT data correction module, after the reference point correction has been performed, corrects coordinates of the CT data while referring to the corrected coordinates of the reference plate.

12. The system of claim 9, wherein the CT data correction module corrects coordinate information of the CT data in which the coordinates of the reference plate have been corrected while referring to a location relationship between the reference plate and the STL data.

13. The system of claim 12, wherein the CT data correction module resizes distances between the coordinates in response to the correction of the coordinate information.

14. The system of claim 9, further comprising a hybrid image generation module configured to generate a 3D image by incorporating an exoskeleton structure of the STL data into the corrected CT data.
